# EUROPEAN PATENT APPLICATION

(11) **EP 2 818 467 A1**
(43) Date of publication of application: **31.12.2014**
(21) Application number: 13173930.2
(22) Date of filing: 27.06.2013
(51) Int. Cl.: C07D 401/12, C07D 219/10, C07D 221/18

(54) **Multi-target drug compounds for the treatment of neurodegenerative disorders**

(71) Applicant: Universitat de Barcelona, 08028 Barcelona (ES); Fundació Institut d'Investigació Biomèdica de Bellvitge, 08908 L'Hospitalet de Llobregat (ES)
(72) Inventor: Muñoz-Torrero López-Ibarra, Diego, 08028 Barcelona (ES); Ferrer Abizanda, Isidre, 08028 Barcelona (ES); Sola Lao, Irene, 08028 Barcelona (ES); Aso Pérez, Ester, 08908 L'Hospitalet de Llobregat (ES)

(57) **Abstract**

The compounds of formula (I), or its pharmaceutically acceptable salts, or any stereoisomer or mixture thereof, wherein: R₁ and R₁' are both H or R₁ and R₁' are taken together forming a biradical of formula -CH₂-C(R₄)=CH-, R₂ and R₃ are radical independently selected from the group consisting of H, F, Cl and (C₁-C₄)-alkyl; R₄ is a (C₁-C₂)-alkyl radical; and n is an integer from 2 to 15, are useful for the treatment of a neurodegenerative disorder such as Alzheimer's disease.

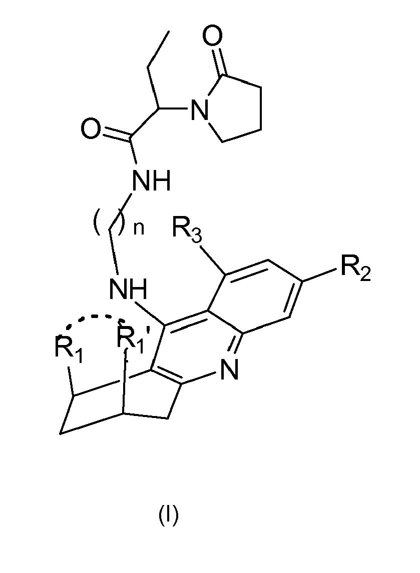

## Description

The invention refers to compounds that are able to modulate the initiating, propagating and final steps of the neurodegenerative process of Alzheimer's disease and to a process for their preparation. It also relates to pharmaceutical compositions comprising these compounds and to their use for the treatment of neurodegenerative disorders such as Alzheimer's disease.

### BACKGROUND ART

Populations in developed countries are growing increasingly older along with medical and social advances. However, healthy ageing can be compromised by the increased susceptibility of elderly people to a number of important diseases that occur late in life, leading to a reduced quality of life, disability and death. Particularly important among these diseases of ageing are neurodegenerative diseases.

Neurodegenerative disorders can be defined as chronic and progressive disorders of the nervous system affecting neurologic and behavioral functions, which start with specific biochemical changes that ultimately lead to distinct histopathologic and clinical syndromes. Among such disorders are Alzheimer's disease (AD), Huntington's disease and Parkinson's disease.

AD is a slow, progressive, and ultimately fatal neurodegenerative disorder, clinically characterized by a noticeable cognitive decline defined by a loss of memory and learning ability, together with a reduced ability to perform basic activities of daily living and a diverse array of neuropsychiatric symptoms. It mainly affects older people. After 65, the likelihood of developing AD doubles every 5 years. Some 35 million people worldwide, i.e. about 0.5% of the world's total population, are suffering dementia, in most cases AD. AD is among the top ten leading causes of death in developed countries, but the only one among them that cannot be cured, prevented or even slowed down. As a consequence of its increasing prevalence and devastating effects, AD is currently affecting every health system in the world. The total worldwide estimated cost associated with AD accounts for approximately 1% of the world's gross domestic product. Worryingly, the figures associated to AD are rising both in terms of mortality and prevalence. Thus, death rates from other major diseases have dropped since 2000, whereas the number of deaths from AD has risen 66%. Also, it is estimated that the prevalence of AD will double every 20 years, reaching 66 million people worldwide by 2030 and 115 million by 2050.

Therapeutic interventions currently available for AD, namely the acetylcholinesterase (AChE) inhibitors donepezil, galantamine and rivastigmine and the glutamate NMDA receptor antagonist memantine, are regarded as merely symptomatic. These drugs compensate for neurotransmitter deficits that arise at the end of the neurotoxic cascade of AD as a consequence of the neuronal death and are responsible for the cognitive decline of AD patients. In contrast, most drug candidates under clinical development have been designed to hit one of the biological targets involved in the early pathogenic events of the neurotoxic cascade of AD that are responsible for the neuronal death, mainly the formation of the beta-amyloid peptide (Aβ), which involves the proteolysis of the amyloid precursor protein by the sequential action of the enzymes β-secretase (BACE-1) and γ-secretase, and the subsequent aggregation of Aβ into toxic oligomers and fibrils. Disappointingly, the most advanced drug candidates in clinical trials hitting these biological targets have failed in the past years. The clinical development of the Aβ-antiaggregating compound tramiprosate and the γ-secretase modulator tarenflurbil, whose launching was expected by 2009/2010, was discontinued in advanced Phase III trials shortly before those dates due to efficacy issues. Analogously, clinical development of the promising BACE-1 inhibitor LY2811376 was discontinued in Phase I trials in 2011 due to toxicity findings observed in parallel longer-term preclinical studies in mice.

It is becoming increasingly apparent that diseases, in general, and complex diseases as AD, in particular, are not straighforward processes but rather complex networks of interconnected protein targets, with alternative, redundant, compensatory signaling pathways that render ineffective the modulation of a single target of the pathological network by compounds as those anti-Alzheimer drug candidates that are recently failing in clinical trials. Conversely, a multi-target approach based on the simultaneous modulation of several targets involved in the pathological network should result in a more efficient therapeutic intervention. Thus, simultaneous blockade of several important nodes of the pathological network of AD is emerging as a promising therapeutic approach for efficiently interfering the underlying mechanisms of the disease.

The multi-target approach to AD can be ideally accomplished by means of single compounds that are structurally suited to hit different biological targets, i.e. one medicine containing a single drug with the ability to hit two or more biological targets. Multi-target drugs can be rationally designed by combination of structural fragments or units of carefully selected compounds that are known to be active toward a particularly important target or pathogenic process.

In vitro inhibitory activities of AChE and AChE-induced Aβ aggregation have been reported for different families of dual binding site AChEIs. The catalytic site of AChE is involved in the hydrolysis of the neurotransmitter acetylcholine, whereas the peripheral site is involved in a binding process with Aβ that accelerates the aggregation of this peptide. Thus, simultaneous blockade of the catalytic and peripheral sites of AChE by dual binding site AChE inhibitors (AChEIs) results in the simultaneous modulation of two important targets of AD pathology, namely Aβ aggregation and the cholinergic deficit. Among the compounds disclosed having in vitro inhibitory activities of AChE and AChE-induced Aβ aggregation are memoquin (cf. e.g. A. Cavalli et al., "A Small Molecule Targeting the Multifunctional Nature of Alzheimer's Disease" Angew. Chem. Int. Ed. 2007, vol. 46, pp. 3689-3692) or some hybrid compounds having the 5,6-dimethoxy-2-[(4-piperidinyl)methyl)]-1-indanone moiety of donepezil (or the indane derivative thereof) and a unit of tacrine or huprine as the peripheral to mid-gorge and active site interacting moieties (cf. WO2007/122274 or WO2011/076969, respectively).

Only very few examples of rationally designed multi-target drug candidates have entered clinical trials for AD, i.e. NP-61 (Phase I, Noscira), for which a potent AChE inhibitory activity and Aβ-antiaggregating effect have been reported, and ladostigil (Phase II, Avraham Pharmaceuticals Ltd.), which in preclinical studies exhibited a multipotent pharmacological profile, it being able to inhibit AChE, prevent the development of memory impairment in aged rats and rescue animal models of AD from memory impairment, afford antidepressant activity and reduce the number of activated inflammatory cells in brain.

Notwithstanding the extensive research efforts invested in the past, no drug effective for preventing, halting or slowing down AD progression has reached the market so far. Therefore, there is currently a very important and acute need to find compounds for the effective treatment of AD. In this context, multi-target drugs hitting the underlying mechanisms of AD seem to be the more realistic approach.

AD has been associated with increased cerebral levels of Aβ, cognitive decline, and also with an increased incidence of unprovoked seizures. High levels of Aβ have been shown to be responsible for eliciting epileptiform activity and seizures in transgenic mice expressing human APP (hAPP), even at early stages of the disease process, this aberrant excitatory neuronal activity contributing causally to learning and memory deficits in these transgenic mice. It has been suggested that Aβ peptides might account for the cognitive deficits in AD patients by eliciting a similar aberrant excitatory neuronal activity also in humans. Therefore both the increased levels of Aβ peptides and the immediately resulting aberrant excitatory neural network activity would constitute primary upstream events in the neurotoxic cascade of AD, that eventually would lead to cognitive decline.

### SUMMARY OF THE INVENTION

Inventors have provided with novel compounds that interfere primary upstream events of the neurotoxic cascade of AD by simultaneously reducing brain Aβ levels as well as Aβ-induced aberrant excitatory neuronal activity, and, therefore, with potential to protect the brain against the deleterious effects of Aβ first on synaptic integrity and eventually on cognition. Indeed, in preliminary *in vivo* studies inventors have found that these compounds reduce brain amyloid burden as well as Aβ-induced astrocytic and microglial response and rescue memory impairment of a transgenic mouse model of AD, namely the APP/PS1 mice.

These effects are especially relevant since if administered in an early phase these compounds might halt the neurodegenerative process, i.e. they should be able to interfere upstream in the neurotoxic cascade of AD, and therefore, positively modify AD progression, which makes these compounds very promising disease modifying anti-Alzheimer drugs.

In addition, the therapy with a single multi-target drug is advantageous over other multi-target therapies that are based on the use of more than one compound, namely drug cocktails (two or more medicines each one containing one different drug) or fixed dose combinations (one medicine containing two or more different drugs), because it results in better patient compliance, no risk for drug-drug interactions, less side-effects, simpler and more predictable pharmacokinetics, clinical studies, regulatory approval and registration procedures, and easier manufacture and formulation as well.

Accordingly, a first aspect of the present invention relates to a compound of formula (I) or its pharmaceutically acceptable salts, or any stereoisomer or mixture of stereoisomers, either of the compound of formula (I) or of their salts, 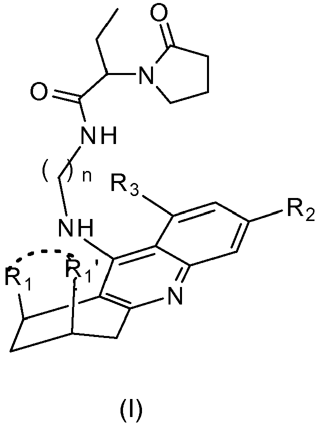 where: R₁ and R₁' are both H or R₁ and R₁' are taken together 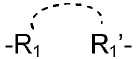 forming a biradical of formula -CH₂-C(R₄)=CH-; R₂ and R₃ are radicals independently selected from the group consisting of H, F, Cl and (C₁-C₄)-alkyl; R₄ is a (C₁-C₂)-alkyl radical; and n is an integer from 2 to 15.

Thus, compounds of formula (I) may have one of the following general formulae:

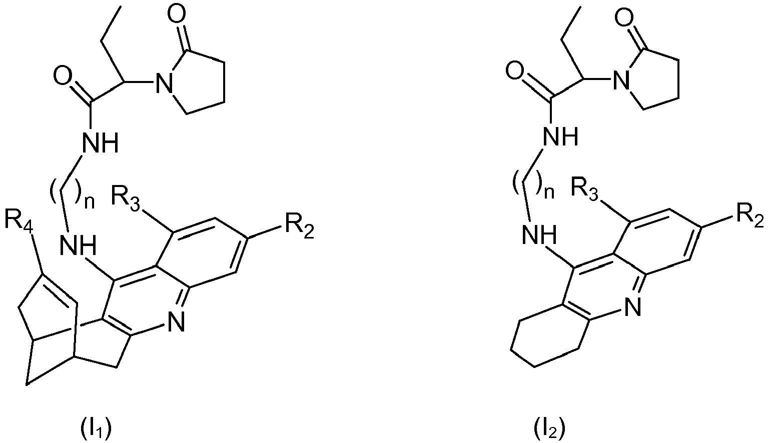

A second aspect of the present invention relates to a process for the preparation of a compound of formula (I) as defined above, or its pharmaceutically acceptable salts, or any stereoisomer or mixture of stereoisomers, either of the compound of formula (I) or of their salts, which comprises: (a) reacting a compound of formula (II), a salt or a reactive derivative thereof, or any stereoisomer or mixtures thereof, either of the compound of formula (II) or of their salts or derivatives, with a compound of formula (III) or a salt thereof, or any stereoisomer or mixtures thereof; wherein R₁, R₁', R₂, R₃, R₄, and n have the same meaning defined above for the compound of formula (I); and, (b) optionally, converting the compound thus obtained into a pharmaceutically acceptable salt by reacting it with the corresponding pharmaceutically acceptable acid.

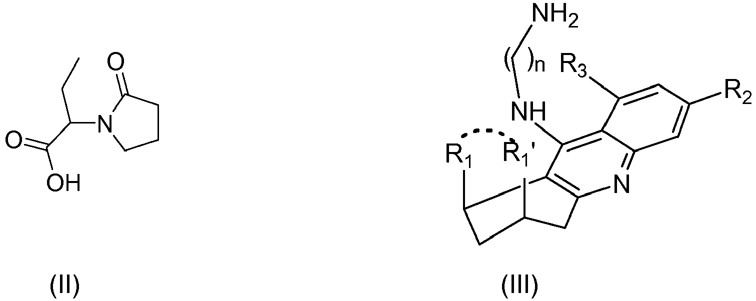

A third aspect of the present invention relates to some new intermediate compounds, in particular to some compounds of formula (III) and of formula (IV) disclosed below,

A fourth aspect of the present invention relates to a pharmaceutical composition comprising a therapeutically effective amount of the compound of formula (I), or a pharmaceutically acceptable salt thereof, including any stereoisomer or mixture thereof, together with appropriate amounts of one or more pharmaceutical excipients or carriers.

A fifth aspect of the invention relates to the compounds of formula (I) as defined above, for use as a medicament.

A sixth aspect of the present invention relates to the compounds of formula (I) as defined above for the prophylactic and/or therapeutic treatment of a neurodegenerative disorder, in particular, Alzheimer's disease.

### BRIEF DESCRIPTION OF THE DRAWINGS

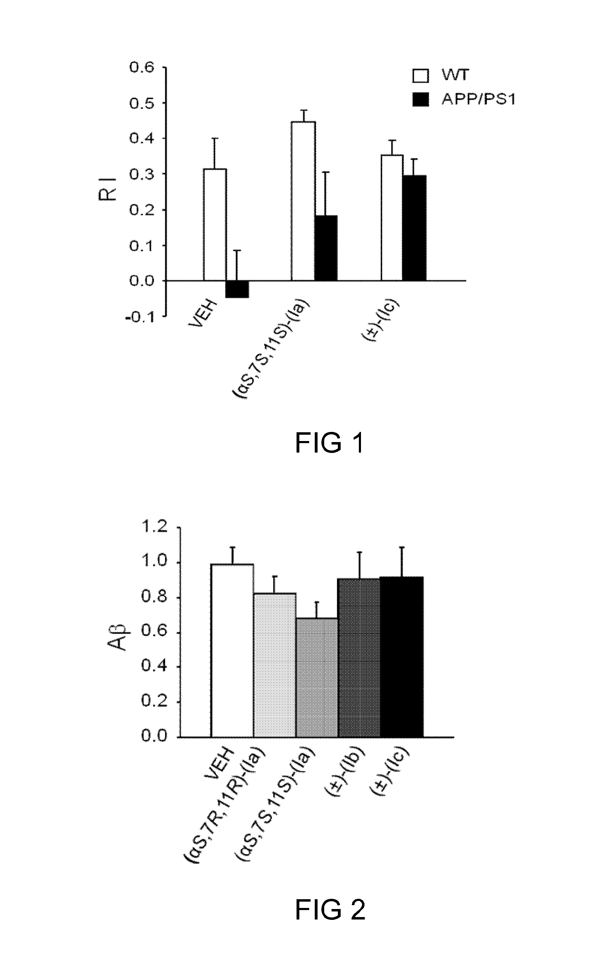

FIG 1 illustrates the effects of compounds of formula (α*S*,7*R*,11*R*)-(Ia), (α*S*,7*S*,11*S*)-(Ia), (±)-(Ib), and (±)-(Ic) on the cognitive failure of APP/PS1 mice, using the two-object recognition test. RI = recognition index; VEH = vehicle; WT = wild-type; APP/PS1 = transgenic mice that express the mutated human amyloid precursor protein (APP) and presenilin 1 (PS1) genes.
FIG 2 illustrates the brain amyloid burden present in APP/PS1 mice treated with compounds of (α*S*,7*R*,11*R*)-(Ia), (α*S*,7*S*,11*S*)-(Ia), (±)-(Ib), and (±)-(Ic), using Aβ immunostaining. Aβ = β-amyloid peptide burden in the cortex of APP/PS1 mice; VEH = vehicle.

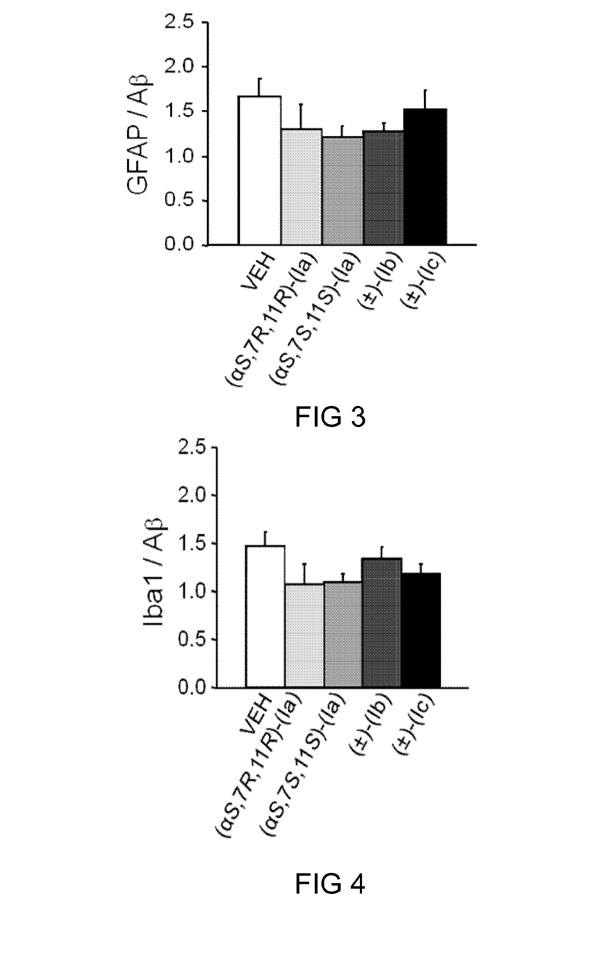

FIG 3 illustrates the level of astrogliosis around amyloid plaques in APP/PS1 mice treated with compounds of (α*S*,7*R*,11*R*)-(Ia), (α*S*,7*S*,11*S*)-(Ia), (±)-(Ib), and (±)-(Ic), using double glial and Aβ immunostaining. GFAP/Aβ = Glial fibrillary acidic protein immunostaining in reference to Aβ plaque area; VEH = vehicle.
FIG 4 illustrates the level of microgliosis around amyloid plaques in APP/PS1 mice treated with compounds of (α*S*,7*R*,11*R*)-(Ia), (α*S*,7*S*,11*S*)-(Ia), (±)-(Ib), and (±)-(Ic), using double glial and Aβ immunostaining. Iba1/Aβ = ionized calcium-binding adapter molecule 1 immunostaining in reference to Aβ plaque area; VEH = vehicle.

### DETAILED DESCRIPTION OF THE INVENTION

As mentioned above, an aspect of the present invention relates to compounds of formula (I), their pharmaceutically acceptable salts, any of their stereoisomers or mixture of stereoisomers, either of the compound of formula (I) or of their salts, wherein: R₁ and R₁' are both H or, alternatively, R₁ and R₁' are taken together and are biradical of formula -CH₂-C(R₄)=CH-; R₂ and R₃ are radicals independently selected from the group consisting of H, F, Cl and (C₁-C₄)-alkyl, for instance, methyl; R₄ is a (C₁-C₂) alkyl radical; and n is an integer from 2 to 15.

Any range given in this document is intended to include the end values of the range.

The new compounds of formula (I) or their salts can exist in solvated, as well as unsolvated forms, including hydrated forms. Thus, they can contain in its structure stoichiometric amounts of solvent in the case of solvates, or of water in the case of hydrates. It is to be understood that the invention encompasses all such solvated, as well as unsolvated forms.

The pharmaceutically acceptable salts of the compound of formula (I) include acid addition salts such as the hydrochloride, but also any other pharmaceutically acceptable salts of other acids such as hydrobromic, hydrofluoric, sulphuric, phosphoric, acetic, citric, fumaric, gluconic, lactic, maleic, succinic or tartaric acid. In a preferred embodiment, the pharmaceutically acceptable salt is the hydrochloride salt.

In a preferred embodiment, compounds of formula (I) are those where R₂ is a radical selected from the group consisting of F, Cl, and methyl; and R₃ is a radical selected from the group consisting of H, F, Cl and methyl.

In another preferred embodiment, compounds of formula (I) are those where R₁ and R₁' are taken together forming a biradical of formula -CH₂-C(R₄)=CH-. In a more preferred embodiment R₄ is methyl. In a still more preferred embodiment, R₂ is Cl. In an even still more preferred embodiment, R₃ is H, Thus, the last compounds of formula (I) have the following formula (I₁ₐ), i.e. compounds of formula (I) with the biradical -CH₂-C(R₄)=CH-, R₂ = Cl, R₃ = H, and R₄ = methyl.

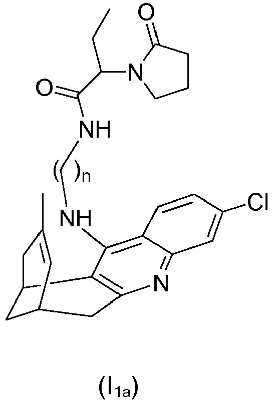

In another preferred embodiment, compounds of formula (I) are those where R₁ and R₁' are H. In a more preferred embodiment, R₂ is Cl or H. In a still more preferred embodiment, R₃ is H. The last compounds may have the following formula (I₂ₐ), i.e. compounds of formula (I) with R₁ = R₁' = R₃ = H and R₂ = Cl ; 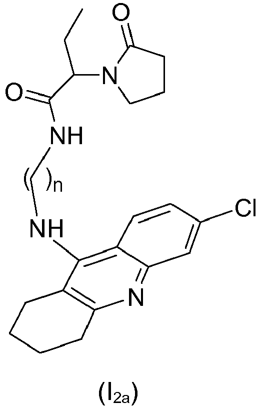 or the formula (I_{2b}), i.e. compounds of formula (I) R₁ = R₁' = R₂ = R₃ = H.

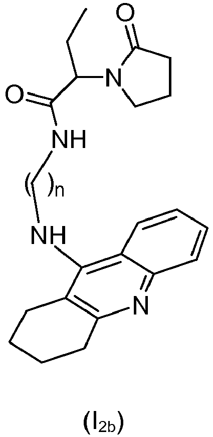

In another preferred embodiment, in combination with one or more features of the various embodiments described above or below, compounds of formula (I) are those where n is an integer from 2 to 12. In another more preferred embodiment, compounds of formula (I) are those where n is an integer from 6 to 8.

The most preferred compounds of formula (I) are those selected from the following list:
(+)-(α*S*,7*R*,11*R*)-*N*-{7-[(3-chloro-6,7,10,11-tetrahydro-9-methyl-7,11-methanocycloocta[*b*]quinolin-12-yl)amino]heptyl}-2-(2-oxopyrrolidin-1-yl)butanamide (α*S*,7*R*,11*R*)-(Ia);
(-)-(α*S*,7*S*,11*S*)-*N*-{7-[(3-chloro-6,7,10,11-tetrahydro-9-methyl-7,11-methanocycloocta[*b*]quinolin-12-yl)amino]heptyl}-2-(2-oxopyrrolidin-1-yl)butanamide (α*S*,7*S*,11*S*)-(Ia);
(±)-*N*-{7-[(6-chloro-1,2,3,4-tetrahydroacridin-9-yl)amino]heptyl}-2-(2-oxopyrrolidin-1-yl)butanamide (±)-(Ib); and
(±)-*N*-{7-[(1,2,3,4-tetrahydroacridin-9-yl)amino]heptyl}-2-(2-oxopyrrolidin-1-yl)butanamide (±)-(Ic).

Compounds of formula (I) as defined above may be prepared by a process which comprises reacting a compound of formula (II), a salt or a reactive derivative thereof, or any stereoisomer or mixture thereof, either of the compound of formula (II) or of their salts or derivatives, 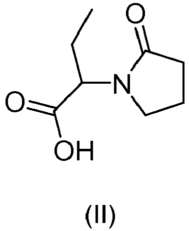 with a compound of formula (III) or a salt thereof, or any stereoisomer or mixture thereof, either of the compound of formula (III) or of their salts, 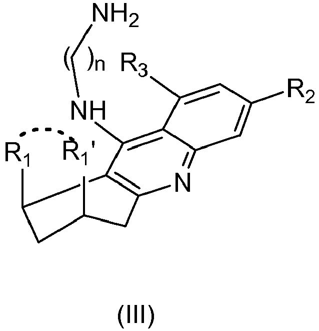 where R₁ and R₁' are both H or R₁ and R₁' are taken together 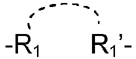 forming a biradical of formula -CH₂-C(R₄)=CH; R₂ and R₃ are radical independently selected from the group consisting of H, F, Cl and (C₁-C₄)-alkyl;
R₄ is a (C₁-C₂) alkyl radical; and n is an integer from 2 to 15; and if desired, the compound thus obtained may be converted into a pharmaceutically acceptable salt by reacting it with the corresponding pharmaceutically acceptable acid.

Reactive derivatives of carboxylic acids of formula (II) include acyl halides, anhydrides, and esters.

The carboxy group of compound (II) can be converted into an acyl halide, such as an acid chloride, for example, by treating the compound (II) with a thionyl halide, such as thionyl chloride.

The carboxyl group of the compound of formula (II) can also be converted into an anhydride, including a mixed anhydride, for example, using a formic acid ester such as a (C₁-C₇)-alkyl formate. In particular, it can be carried out for example by treating a salt of compound (II), such as an ammonium or alkali metal salt, with an haloformic acid ester, in particular a chloroformic acid ester, such as a (C₁-C₇)-alkyl formate.

The carboxy group of compound (II) can also be converted into an activated ester, such as a cyanomethyl ester, a nitrophenyl ester such as a 4-nitrophenyl ester, or a polyhalophenyl ester such as a pentachlorophenyl ester. It can be made by treating the compound of formula (II) with an appropriate hydroxyl compound in the presence of a suitable condensing agent, such as *N,N'*-dicyclohexylcarbodiimide.

Either the compound of formula (II) or the reactive derivative of the types disclosed above can be reacted with an amine of formula (III) and in this way amide compounds of the formula (I) can be obtained directly or via the intermediate compounds describe above.

Other non-activated esters, such as (C₁-C₇)-alkyl esters of compounds of formula (II), which contain, for example, (C₂-C₈)-alkoxycarbonyl as a substituent, can also be brought to reaction with the amines of formula (III). Generally, the reaction is carried out in the presence of a base, preferably the base is selected from an alkaline or alkaline earth metal hydroxide such as sodium or potassium hydroxide, an alkaline or alkaline earth metal carbonate such as sodium or potassium carbonate, and an organic amine, preferably a tertiary amine such as triethylamine.

The pharmaceutically acceptable salts of the compounds of formula (I), in particular, the hydrochlorides, but also other pharmaceutically acceptable salts of other acids such as those mentioned above can be prepared conventionally, e.g. by mixing a solution of the free base compound and the corresponding acid in a suitable solvent, e.g. ethanol and recovering the acid addition salt either as precipitate, or by evaporation of the solution. Salts can also be converted into the free compounds in a customary manner, for example, by treating with a suitable basic agent.

In view of the close relationship between the novel compounds in the free base form and in the form of their salts, in the preceding text and below when it is referred to the compounds (I) may be understood as meaning either the free base compounds or their salts.

As mentioned above, the compounds of formula (I) or their salts can also be obtained in the form of their hydrates or can include other solvents used for crystallization in their lattice. The obtention of solvates and hydrates depends on the solvent used and the crystallization conditions that can be determined by the skilled person. The new compounds herein described have the ability to retain molecules of water, which in some cases cannot be removed after drying the analytical samples at 65 °C/2 Torr for 7 days.

Depending on the choice of the starting materials and processes, the compounds of formula (I) can be present in the form of one of the possible isomers or as mixtures thereof, for example as pure optical isomers, or as isomer mixtures, such as racemates, or stereoisomer mixtures.

Racemates and diastereomer mixtures obtained can be separated into the pure isomers or racemates in a known manner on the basis of the physicochemical differences of the components, for example by fractional crystallization. Racemates obtained may furthermore be resolved into the optical enantiomers by known methods, for example by recrystallization from an optically active solvent, chromatography on chiral adsorbents, with the aid of suitable microorganisms, by cleavage with specific immobilized enzymes, via the formation of inclusion compounds or by conversion into diastereomeric salts. Thus, for example a racemic compound of formula (I) can be separated into its enantiomers by reaction of the racemate with an optically active acid and separation of the diastereomer mixture obtained. Examples of appropriate optically active acids are carboxylic acids such as tartaric or malic acid; sulfonic acids such as camphorsulfonic acid. The separation of the diastereomer mixture obtained in this manner, can be done on the basis of its differing solubilities. Then, the desired enantiomer can be liberated by the action of suitable agents. The most active enantiomer is advantageously isolated.

The separation of a racemic compound into its enantiomers can also be carried out over any of the intermediates used for the preparation of the compounds of formula (I) which contain the huprine fragment, i.e. any of the intermediate compounds (III), (IV), (V), or (VII), where R₁ and R₁' form the biradical -CH₂-C(R₄)=CH- . Preferably, the separation is carried out over the compound of formula (V) below, for instance by chromatography on chiral adsorbents.

The enantiomers of the compound of formula (II) are commercially available.

Compounds of formula (III) or their salts or their stereoisomers can be prepared by a process which comprises submitting a compound of formula (IV) or a salt thereof, or any stereoisomer or mixture thereof, either of the compound of formula (IV) or of their salts, to a reduction reaction, 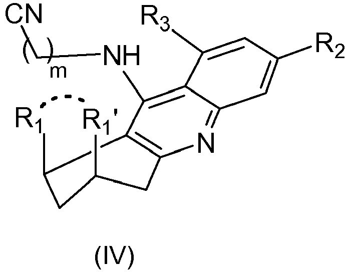 where: R₁ and R₁' are both H or, alternatively, R₁ and R₁' are taken together 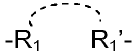 forming a biradical of formula -CH₂-C(R₄)=CH-; R₂ and R₃ are radicals independently selected from the group consisting of H, F, Cl and (C₁-C₄)-alkyl; R₄ is a (C₁-C₂)-alkyl radical; and m is an integer from 1 to 14. Preferably, the (C₁-C₄)-alkyl is methyl.

The reduction reaction can be carried out using a reducing agent, for instance lithium aluminum hydride in the presence of a suitable solvent, for instance, a (C₂-C₆)-ether such as diethyl ether or tetrahydrofuran.

Compounds of formula (IV) or or can bye Compounds of formula (IV) or their salts or their stereoisomers can be (V) prepared by a process which comprises reacting a compound of formula (V) or a salt thereof, or any stereoisomer or mixture thereof, either of the compound of formula (V) or of their salts, with a compound of formula (VI). In compound of formula (V), R₁, R₁', R₂, and R₃ have the same meaning as for compound of formula (III), and in compound of formula (VI) m is an integer from 1 to 14 and X is an halogen. Preferably the halogen is Cl, Br or I, more preferably, the halogen is Br.

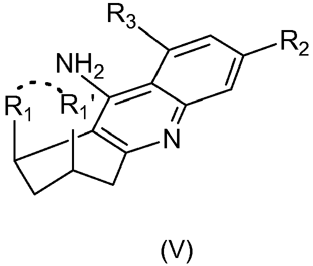

X-(CH₂)ₘ-CN (VI)

Alternatively, compounds of formula (III) or their salts or theirs stereoisomers can be prepared by a process which comprises reacting a compound of formula (VII) or any stereoisomer or mixture thereof, with a compound of formula (VIII). In compound of formula (VII), R₁, R₁', R₂, and R₃ have the same meaning as for compound of formula (III), and in compound of formula (VIII), n is an integer from 2 to 15.

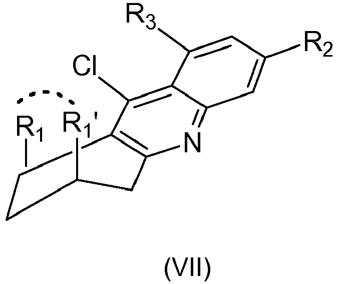

H₂N-(CH₂)ₙ-NH₂ (VIII)

The preferred values of R_{1,} R₁', R_{2,} R₃, for the compound of formula (I) are also preferred values for the compounds of formula (III), (IV), (V), and (VII) in the preparation process of the invention. The preferred values of n for the compound of formula (I) are also preferred values for the compound of formula (III). Preferred values of m in compound of formula (IV) are comprised from 1 to 11, more preferred are the ones comprised from 5 to 7.

Some intermediate compounds of formula (III) and of formula (IV) are new and also form part of the invention.

Thus, it is part of the invention the provision of an intermediate compound of formula (III) or their salts, or any stereoisomer or mixture thereof,
wherein: R₁ and R₁' are taken together 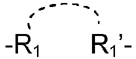 forming a biradical of formula -CH₂-C(R₄)=CH-; R₂ and R₃ are radicals independently selected from the group consisting of H, F, Cl and (C₁-C₄)-alkyl;
R₄ is a (C₁-C₂)-alkyl radical; and n is an integer from 2 to 7. Preferably, the (C₁-C₄)-alkyl is a methyl.

It is also part of the invention the provision of an intermediate compound of formula (IV) or their salts, or any stereoisomer or mixture thereof, where both R₁ and R₁' are H; n is an integer from 2 to 15; and R₂ and R₃ are radicals independently selected from the group consisting of H, F, Cl and (C₁-C₄)-alkyl; or alternatively, R₁ and R₁' are taken together 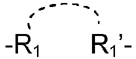 forming a biradical of formula -CH₂-C(R₄)=CH-; R₂ and R₃ are radicals independently selected from the group consisting of H, F, Cl and (C₁-C₄)-alkyl; R₄ is a (C₁-C₂)-alkyl radical; and m is an integer from 1 to 6. Preferably, the (C₁-C₄)-alkyl is a methyl.

In a preferred embodiment, compounds of formula (III) or (IV) are those where R₂ is a radical selected from the group consisting of F, Cl and methyl; and R₃ is a radical selected from the group consisting of H, F, Cl and methyl. The other preferred embodiments described above for the values of R_{1,} R₁', R₂, R₃ and R₄ for the compound of formula (I) are also preferred embodiments for the compounds of formula (III) and (IV).

As mentioned above it is also part of the invention the pharmaceutical composition comprising a therapeutically effective amount of the compound of formula (I), or a pharmaceutically acceptable salt thereof, including any stereoisomer or mixture thereof, together with appropriate amounts of one or more pharmaceutical excipients or carriers.

The term "therapeutically effective amount" as used herein, refers to the amount of a compound that, when administered, is sufficient to prevent development of, or alleviate to some extent, one or more of the symptoms of a neurodegenerative disorder, in particular, Alzheimer's disease. The particular dose of compound administered according to this invention will of course be determined by the particular circumstances surrounding the case, including the compound administered, the route of administration, the particular condition being treated, and the similar considerations.

The term "pharmaceutical composition" refers to a mixture of a compound disclosed herein with other chemical components, such as diluents or carriers. The pharmaceutical composition facilitates administration of the compound to an organism.

The terms "pharmaceutically acceptable excipients or carriers" refer to pharmaceutically acceptable material, composition or vehicle, such as liquid or solid filler, diluent, excipient, solvent, or encapsulating material. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "treatment" meant to include alleviating or eradicating a disorder, disease, or condition, or one or more of the symptoms associated with the disorder, disease or condition, or alleviating or eradicating the cause(s) of the disorder, disease, or condition itself.

Another aspect of the invention relates to the compounds of formula (I) as defined above, for use as a medicament.

A further aspect of the present invention relates to the compounds of formula (I) as defined above for use in the prophylactic and/or therapeutic treatment of a neurodegenerative disorder, in particular, in a mammal, including a human. This aspect can also be formulated as the use of the compounds of formula (I) as defined above, for the preparation of a medicament for the prophylactic and/or therapeutic treatment of a neurodegenerative disorder, in particular in a mammal, including a human. The invention also relates to a method of treatment and/or prophylaxis of a mammal, including a human, suffering from or being susceptible to a neurodegenerative disorder, said method comprises the administration to said patient of a therapeutically effective amount of the compound of formula (I) as defined above, together with pharmaceutically acceptable excipients or carriers.

Particularly, the neurodegenerative disorder is a neurodegenerative disorder with abnormal protein aggregates.

Examples of neurodegenerative disorders with abnormal protein aggregates include β-amyloidopathies (e.g. Alzheimer's disease), tauopathies (e.g. Alzheimer's disease, Pick's disease, progressive supranuclear palsy, argyrophilic grain disease, frontotemporal lobar degeneration), synucleinopathies (e.g. Parkinson's disease, dementia with Lewy bodies, multiple system atrophy), polyglutamine diseases (i.e. Huntington's disease), prion diseases (e.g. Creutzfeldt-Jakob disease, kuru, Gerstmann-Sträussler-Scheinker syndrome, fatal familial insomnia) and TDP-43 proteinopathies (i.e. frontotemporal lobar degeneration, amyotrophic lateral sclerosis, Perry syndrome).

In a preferred embodiment, the neurodegenerative disorder is Alzheimer's disease.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Chemistry General Methods.

Melting points were determined in open capillary tubes with a MFB 595010M Gallenkamp melting point apparatus.

Elemental analyses and high resolution mass spectra were carried out at the Mycroanalysis Service of the IIQAB (CSIC, Barcelona, Spain) with a Carlo Erba model 1106 analyzer, and at the Centres Científics i Tecnològics of the University of Barcelona with a LC/MSD TOF Agilent Technologies spectrometer.

IR spectra were run on a Perkin-Elmer Spectrum RX I spectrophotometer. Absorption values are expressed as wave-numbers (cm⁻¹); only significant absorption bands are given.

Column chromatography was performed on silica gel 60 AC.C (40-60 mesh, SDS, ref 2000027). Thin-layer chromatography was performed with aluminum-backed sheets with silica gel 60 F254 (Merck, ref 1.05554), and spots were visualized with UV light and 1% aqueous solution of KMnO4.

Analytical grade solvents were used for crystallization, while pure for synthesis solvents were used in the reactions, extractions and column chromatography.

For characterization purposes, the new hybrids were transformed into the corresponding hydrochlorides, and recrystallized. The analytical samples of all of the new hybrids which were subjected to pharmacological evaluation possess a purity ≥95% as evidenced by their elemental analyses. Worthy of note, as previously reported for some tacrine-related dimeric compounds, the new hybrids herein described have the ability to retain molecules of water, which cannot be removed after drying the analytical samples at 65 °C/2 Torr for 7 days. Thus, the elemental analyses of these compounds showed the presence of variable amounts of water, which have been indicated in the corresponding compound formulas.

### Example 1: Preparation of (+)-(7R,11R)-7-[(3-chloro-6,7,10,11-tetrahydro-9-methyl-7,11-methanocycloocta[b]quinolin-12-yl)amino]heptanenitrile (+)-(IVa)

A well stirred suspension of (+)-huprine Y (+)-(Va) (1.00 g, 3.52 mmol) and finely powdered NaOH (0.46 g, 11.6 mmol) in anhydrous DMSO (15 mL) was heated every 10 min approximately with a heat gun for 1 h and at room temperature for one additional hour, and then a solution of 7-bromoheptanenitrile (VIa) (90% purity, 0.61 mL, 0.77 g, 4.05 mmol) in DMSO (1 mL) was added dropwise for 30 min. The reaction mixture was stirred at room temperature overnight, diluted with aqueous 2 N NaOH (50 mL) and water (50 mL) and extracted with AcOEt (3 x 75 mL). The combined organic extracts were washed with water (3 x 100 mL) and with 5 N NaOH (6 x 100 mL), dried with anhyd. Na₂SO₄, filtered and evaporated under reduced pressure to give a yellow oil (780 mg), which was subjected to column chromatography [40-60 µm silica gel (23 g), CH₂Cl₂/50% aq NH₄OH 100:0.2], to provide the compound of the title (156 mg) and an impure product. The impure fraction was subjected to column chromatography [40-60 µm silica gel (28 g), CH₂Cl₂/MeOH/50% aq NH₄OH mixtures], to provide the compound of the title (599 mg, 55% total yield) as a yellowish solid. The isolated compound of the title was transformed into the corresponding hydrochloride as follows: A solution of the free base (40 mg, 0.10 mmol) in CH₂Cl₂ (3 mL) was filtered through a 0.2 µm NYL filter and treated with a 0.53 N methanolic solution of HCl (0.58 mL, 0.31 mmol). The solution was concentrated *in vacuo* to dryness and the solid residue was washed with pentane (3 x 2 mL) and dried at 65 °C/2 Torr for 7 days to give (+)-(IVa)•HCl (51 mg) as a yellowish solid. Characterization: (+)-(IVa): R_{f} = 0.80 (CH₂Cl₂/MeOH/50% aq NH₄OH 9:1:0.05). (+)-(IVa)•HCl: mp 107-110 °C (CH₂Cl₂/MeOH 84:16); [α]²⁰_{D} = +234 (*c* = 0.52, MeOH); IR (KBr) v: 3500-2500 (max at 3229, 3044, 2925, 2854, 2775, 2652, N-H,⁺N-H, and C-H st), 2242 (CN st), 1631, 1581, 1560, 1513 (ar-C-C and ar-C-N st) cm⁻¹; HRMS (ESI) calcd for (C₂₄H₂₈³⁵ClN₃ + H⁺) 394.2045, found 394.2046.

### Example 2: Preparation of (-)-(7S,11S)-7-[(3-chloro-6,7,10,11-tetrahydro-9-methyl-7,11-methanocycloocta[b]quinolin-12-yl)amino]heptanenitrile (-)-(IVa)

It was prepared as described for the compound of Example 1. From (-)-huprine Y (- )-(Va) (1.00 g, 3.52 mmol) and a solution of 7-bromoheptanenitrile (VIa) (90% purity, 0.61 mL, 0.77 g, 4.05 mmol) in anhydrous DMSO (15 mL), a yellow oily residue (953 mg) was obtained and subjected to column chromatography [40-60 µm silica gel (30 g), CH₂Cl₂/MeOH/50% aq NH₄OH mixtures]. On elution with CH₂Cl₂/MeOH/50% aq NH₄OH 97.5:2.5:0.2, the compound of the title (514 mg, 52% yield) was isolated as a yellowish solid. Characterization: (-)-(IVa): R_{f} = 0.80 (CH₂Cl₂/MeOH/50% aq NH₄OH 9:1:0.05). (-)-(IVa)•HCl: mp 98-102 °C (CH₂Cl₂/MeOH 84:16); [α]²⁰_{D} = -203 (*c* = 1, MeOH); IR (KBr) v: 3500-2500 (max at 3229, 3044, 2928, 2905, 2865, 2759, 2649, N-H,⁺N-H, and C-H st), 2234 (CN st), 1630, 1582, 1567,1513 (ar-C-C and ar-C-N st) cm⁻¹; HRMS (ESI) calcd for (C₂₄H₂₈³⁵ClN₃ + H⁺) 394.2045, found 394.2049.

### Example 3. Preparation of 7-[(6-chloro-1,2,3,4-tetrahydroacridin-9-yl)amino]heptanenitrile (IVb)

It was prepared as described for the compound of Example 1. From 6-chlorotacrine (Vb) (2.00 g, 8.60 mmol) and a solution of 7-bromoheptanenitrile (VIa) (90% purity, 1.55 mL, 1.96 g, 10.3 mmol) in anhydrous DMSO (20 mL), a yellow oily residue (3.06 g) was obtained and subjected to column chromatography [40-60 µm silica gel (92 g), CH₂Cl₂/50% aq NH₄OH 100:0.2], to provide the compound of the title (2.06 g, 70% yield) as a yellowish solid. Characterization: (IVb): R_{f} = 0.92 (CH₂Cl₂/MeOH/50% aq NH₄OH 9:1:0.05). (IVb)•HCl: mp 86-87 °C (AcOEt/MeOH 71:29); IR (KBr) v: 3500-2500 (max at 3347, 3138, 3059, 2928, 2858, 2744, N-H,⁺N-H, and C-H st), 2245 (CN st), 1639, 1605, 1573, 1524 (ar-C-C and ar-C-N st) cm⁻¹; HRMS (ESI) calcd for (C₂₀H₂₄³⁵ClN₃ + H⁺) 342.1732, found 342.1737.

### Example 4. Preparation of 7-[(1,2,3,4-tetrahydroacridin-9-yl)amino]heptanenitrile (IVc)

It was prepared as described for the compound of Example 1. From tacrine (Vc) (1.70 g, 8.60 mmol) and a solution of 7-bromoheptanenitrile (VIa) (90% purity, 1.55 mL, 1.96 g, 10.3 mmol) in anhydrous DMSO (20 mL), a yellow oily residue (2.53 g) was obtained and subjected to column chromatography [40-60 µm silica gel (76 g), CH₂Cl₂/50% aq NH₄OH 100:0.2], to provide the compound of the title (1.75 g, 67% yield) as a yellowish sticky solid. Characterization: (IVc): R_{f} = 0.77 (CH₂Cl₂/MeOH/50% aq NH₄OH 9:1:0.05). (IVc)•HCl: IR (KBr) v: 3500-2500 (max at 3237, 3126, 3054,3023, 2932, 2860, 2770, 2713, N-H,⁺N-H, and C-H st), 2242 (CN st), 1633, 1586, 1573, 1523 (ar-C-C and ar-C-N st) cm⁻¹; HRMS (ESI) calcd for (C₂₀H₂₅N₃ + H⁺) 308.2121, found 308.2118.

### Example 5: Preparation of (+)-(7R,11R)-7-[(3-chloro-6,7,10,11-tetrahydro-9-methyl-7,11-methanocycloocta[b]quinolin-12-yl)amino]heptan-1-amine (+)-(IIIa)

A suspension of nitrile (+)-(IVa) (599 mg, 1.52 mmol) in anhyd. Et₂O (29 mL) was cooled to 0 °C with an ice bath and treated dropwise with a 4 M solution of LiAlH₄ in Et₂O (1.52 mL, 6.08 mmol). The reaction mixture was warmed to room temperature, stirred overnight, cooled to 0 °C and treated dropwise with a 4 M solution of LiAlH₄ in Et₂O (0.80 mL 3.20 mmol). The resulting mixture was warmed to room temperature, stirred for 20 h, and treated dropwise with water and aq 5 N NaOH, and extracted with AcOEt (3 x 100 mL). The combined organic extracts were washed with water (3 x 100 mL), dried with anhyd. Na₂SO₄, filtered, and evaporated under reduced pressure to give a crude product (445 mg), which was subjected to column chromatography [40-60 µm silica gel (10 g), CH₂Cl₂/MeOH/50% aq NH₄OH mixtures]. On elution with CH₂Cl₂/MeOH/50% aq NH₄OH 99:1:1, the compound of the title (270 mg, 45% yield) was isolated as a yellowish oil. The isolated compound of the title was transformed into the corresponding dihydrochloride as follows: A solution of the free base (40 mg, 0.10 mmol) in CH₂Cl₂ (10 mL) was filtered through a 0.2 µm NYL filter and treated with a 0.53 N methanolic solution of HCl (1.71 mL, 0.91 mmol). The solution was concentrated *in vacuo* to dryness and the solid residue was washed with pentane (3 x 2 mL) and dried at 65 °C/2 Torr for 7 days to give (+)-(IIIa)•2HCl (45 mg) as a yellowish solid. Characterization: (+)-(IIIa): R_{f} = 0.16 (CH₂Cl₂/MeOH/50% aq NH₄OH 9:1:0.05). (+)-(IIIₐ)•2HCl: mp 179-183 °C (CH₂Cl₂/MeOH 85:15); [α]²⁰_{D} = +176 (*c* = 1.08, MeOH); IR (KBr) v: 3500-2500 (max at 3240, 2923, 2849, 2802, N-H,⁺N-H, and C-H st), 1629, 1581, 1551, 1513 (ar-C-C and ar-C-N st) cm⁻¹; HRMS (ESI) calcd for (C₂₄H₃₂³⁵ClN₃ + H⁺) 398.2357, found 398.2356.

### Example 6: Preparation of (-)-(7S,11S)-7-[(3-chloro-6,7,10,11-tetrahydro-9-methyl-7,11-methanocycloocta[b]quinolin-12-yl)amino]heptan-1-amine (-)-(IIIa)

It was prepared as described for the compound of Example 5. From nitrile (-)-(IVa) (474 mg, 1.2 mmol), the compound of the title (432 mg, 91 % yield) was directly obtained as a yellowish oil, without the need for a chromatographic purification. Characterization: (-)-(IIIa): R_{f} = 0.16 (CH₂Cl₂/MeOH/50% aq NH₄OH 9:1:0.05). (-)-(IIIa)•2HCl: mp 176-180 °C (CH₂Cl₂/MeOH 85:15); [α]²⁰_{D} = -197 (*c* = 1.14, MeOH); IR (KBr) v: 3500-2500 (max at 3250, 2891, 2870, 2638, N-H,⁺N-H, and C-H st), 1629, 1580, 1548, 1538, 1514 (ar-C-C and ar-C-N st) cm⁻¹; HRMS (ESI) calcd for (C₂₄H₃₂³⁵ClN₃ + H⁺) 398.2357, found 398.2356.

### Example 7: Preparation of 7-[(6-chloro-1,2,3,4-tetrahydroacridin-9-yl)amino]heptan-1-amine (IIIb)

It was prepared as described for the compound of Example 5. From nitrile (IVb) (2.06 g, 6.03 mmol), a crude product (1.69 g) was obtained and subjected to column chromatography [40-60 µm silica gel (20 g), CH₂Cl₂/MeOH/50% aq NH₄OH mixtures]. On elution with CH₂Cl₂/MeOH/50% aq NH₄OH 100:0.5:0.2 to 95:5:0.2, the compound of the title (602 mg, 30% yield) was isolated as a yellowish oil. Characterization: (IIIb): R_{f} = 0.12 (CH₂Cl₂/MeOH/50% aq NH₄OH 9:1:0.05). (IIIb)•2HCl: IR (KBr) ν: 3500-2500 (max at 3379, 3252, 2928, 2852, 2795, 2661 N-H,⁺N-H, and C-H st), 1630, 1573, 1514 (ar-C-C and ar-C-N st) cm⁻¹.

### Example 8: Preparation of 7-[(1,2,3,4-tetrahydroacridin-9-yl)amino]heptan-1-amine (IIIc)

It was prepared as described for the compound of Example 5. From nitrile (IVc) (1.75 g, 5.70 mmol), a crude product (1.69 g) was obtained and subjected to column chromatography [40-60 µm silica gel (40 g), CH₂Cl₂/MeOH/50% aq NH₄OH mixtures]. On elution with CH₂Cl₂/MeOH/50% aq NH₄OH 80:20:0.2, the compound of the title (810 mg, 46% yield) was isolated as a yellowish oil. Characterization: (IIIc): R_{f} = 0.10 (CH₂Cl₂/MeOH/50% aq NH₄OH 9:1:0.05). (IIIc)•2HCl: IR (KBr) ν: 3500-2500 (max at 3374, 3264, 2930, 2857 N-H,⁺N-H, and C-H st), 1633, 1584, 1573, 1522 (ar-C-C and ar-C-N st) cm⁻¹.

### Example 9: Preparation of (+)-(αS,7R,11R)-N-{7-[(3-chloro-6,7,10,11-tetrahydro-9-methyl-7,11-methanocycloocta[b]quinolin-12-yl)amino]heptyl}-2-(2-oxopyrrolidin-1-yl)butanamide (αS,7R,11R)-(Ia)

A solution of (*S*)-2-(2-oxopyrrolidin-1-yl)butanoic acid (IIa) (99 mg, 0.58 mmol) in anhyd. CH₂Cl₂ (6 mL) was cooled to 0 °C with an ice bath, and treated dropwise with anhyd. Et₃N (0.16 mL, 117 mg, 1.16 mmol) and ethyl chloroformate (0.05 mL, 62.8 mg, 0.58 mmol). The resulting mixture was stirred at 0 °C for 30 min and treated with a solution of amine (+)-(7*R*,11*R*)-(IIIa) (230 mg, 0.58 mmol) in anhyd. CH₂Cl₂ (5 mL). The reaction mixture was stirred at room temperature for 3 days and treated with 10% aq Na₂CO₃ (40 mL). The phases were separated and the aqueous phase was further extracted with CH₂Cl₂ (3 x 30 mL). The combined organic extracts were dried with anhyd. Na₂SO₄, filtered, and evaporated under reduced pressure to give a crude product (343 mg), which was subjected to column chromatography [40-60 µm silica gel (10 g), CH₂Cl₂/MeOH/50% aq NH₄OH mixtures]. On elution with CH₂Cl₂/MeOH/50% aq NH₄OH 99.95:0.05:0.2, the compound of the title (176 mg, 56% yield) was isolated as a yellowish solid. The isolated compound of the title was transformed into the corresponding hydrochloride as follows: A solution of the free base (176 mg, 0.32 mmol) in CH₂Cl₂ (15 mL) was filtered through a 0.2 µm NYL filter and treated with a 0.53 N methanolic solution of HCl (2.7 mL, 1.43 mmol). The solution was concentrated *in vacuo* to dryness and the solid residue was washed with pentane and dried at 65 °C/2 Torr for 7 days to give (α*S*,7*R*,11*R*)-(Ia)•HCl (168 mg) as a yellowish solid. Characterization: (α*S*,7*R*,11*R*)-(Ia): R_{f} = 0.70 (CH₂Cl₂/MeOH/50% aq NH₄OH 9:1:0.05). (α*S*,7*R*,11*R*)-(Ia)•HCl: mp 102-105 °C (CH₂Cl₂/MeOH 85:15); [α]²⁰_{D} = +152 (*c* = 0.48, MeOH); IR (KBr) ν: 3500-2500 (max at 3256, 3055, 2925, 2860, 2796, N-H,⁺N-H, and C-H st), 1657, 1649, 1630, 1581, 1565, 1514 (C=O, ar-C-C and ar-C-N st) cm⁻¹; HRMS (ESI) calcd for (C₃₂H₄₃³⁵ClN₄O₂ + H⁺) 551.3147, found 551.3138. Elemental analysis, calcd for (C₃₂H₄₃ClN₄O₂•HCl•0.5H₂O) C 64.42, H 7.60, N 9.39, Cl 11.88; found C 64.86, H 7.98, N 8.64, Cl 11.00.

### Example 10: Preparation of (-)-(αS,7S,11S)-N-{7-[(3-chloro-6,7,10,11-tetrahydro-9-methyl-7,11-methanocycloocta[b]quinolin-12-yl)amino]heptyl}-2-(2-oxopyrrolidin-1-yl)butanamide (αS,7S,11S)-(Ia)

It was prepared as described for the compound of Example 9. From (*S*)-2-(2-oxopyrrolidin-1-yl)butanoic acid (*S*)-(IIa) (169 mg, 0.99 mmol) and amine (-)-(7*R*,11*R*)-(IIIa) (392 mg, 0.99 mmol), a reaction crude (681 mg) was obtained and subjected to column chromatography [40-60 µm silica gel (20 g), CH₂Cl₂/MeOH/50% aq NH₄OH mixtures]. On elution with CH₂Cl₂/MeOH/50% aq NH₄OH 99.95:0.05:0.2, the compound of the title (30 mg) and an impure fraction (271 mg) were isolated. The impure product was subjected to column chromatography [40-60 µm silica gel (10 g), hexane/AcOEt/Et₃N mixtures]. On elution with hexane/AcOEt/Et₃N 30:70:0.2 to 10:90:0.2, the compound of the title (131 mg, 34% total yield) was isolated as a yellowish solid. Characterization: (α*S*,7*S*,11*S*)-(Ia): R_{f}= 0.71 (CH₂Cl₂/MeOH/50% aq NH₄OH 9:1:0.05). (α*S*,7*S*,11*S*)-(Ia)•HCl: mp 96-99 °C (H₂Cl₂/MeOH 83:17); [α]²⁰_{D} = -195 (*c* = 0.80, MeOH); IR (KBr) ν: 3500-2500 (max at 3249, 3055, 2925, 2854, 2786, N-H,⁺N-H, and C-H st), 1656, 1649, 1633, 1581, 1565, 1518 (C=O, ar-C-C and ar-C-N st) cm⁻¹; HRMS (ESI) calcd for (C₃₂H₄₃³⁵ClN₄O₂ + H⁺) 551.3147, found 551.3135. Elemental analysis, calcd for (C₃₂H₄₃ClN₄O₂•HCl•H₂O) C 63.46, H 7.66, N 9.25, Cl 11.71; found C 63.95, H 7.61, N 8.45, Cl 11.21.

### Example 11: Preparation of (±)-N-{7-[(6-chloro-1,2,3,4-tetrahydroacridin-9-yl)amino]heptyl}-2-(2-oxopyrrolidin-1-yl)butanamide (±)-(Ib)

It was prepared as described for the compound of Example 9. From (±)-2-(2-oxopyrrolidin-1-yl)butanoic acid (±)-(IIa) (298 mg, 1,74 mmol) and (IIIb) (602 mg, 1.74 mmol), a reaction crude (892 mg) was obtained and subjected to column chromatography [40-60 µm silica gel (27 g), CH₂Cl₂/MeOH/50% aq NH₄OH mixtures]. On elution with CH₂Cl₂/MeOH/50% aq NH₄OH 99.8:0.2:0.2 to 99:1:0.2, the compound of the title (677 mg, 78% yield) was isolated as a yellowish solid. Characterization: (±)-(Ib): R_{f}= 0.65 (CH₂Cl₂/MeOH/50% aq NH₄OH 9:1:0.05). (±)-(Ib)•HCl: mp 65-68 °C (CH₂Cl₂/MeOH 86:14); IR (KBr) ν: 3500-2500 (max at 3251, 3071, 3050, 2928, 2854, 2802, N-H,⁺N-H, and C-H st), 1658, 1652, 1632, 1601, 1573, 1538, 1519 (C=O, ar-C-C and ar-C-N st) cm⁻¹; HRMS (ESI) calcd for (C₂₈H₃₉³⁵ClN₄O₂ + H⁺) 499.2834, found 499.2827. Elemental analysis, calcd for (C₂₈H₃₉ClN₄O₂•HCl•H₂O) C 60.75, H 7.65, N 10.12, Cl 12.81; found C 60.91, H 7.81, N 9.87, Cl 12.73.

### Example 12: Preparation of (±)-N-{7-[(1,2,3,4-tetrahydroacridin-9-yl)amino]heptyl}-2-(2-oxopyrrolidin-1-yl)butanamide (±)-(Ic)

It was prepared as described for the compound of Example 9. From (±)-2-(2-oxopyrrolidin-1-yl)butanoic acid (±)-(IIa) (445 mg, 2.60 mmol) and (IIIc) (810 mg, 2,60 mmol), a reaction crude (125 g) was obtained and subjected to column chromatography [40-60 µm silica gel (30 g), CH₂Cl₂/MeOH/50% aq NH₄OH mixtures]. On elution with CH₂Cl₂/MeOH/50% aq NH₄OH 99.8:0.2:0.2 to 98:2:0.2, the compound of the title (1.12 g, 93% yield) was isolated as a yellowish solid. Characterization: (±)-(Ic): R_{f}= 0.52 (CH₂Cl₂/MeOH/50% aq NH₄OH 9:1:0.05). (±)-(Ic)•HCl: mp 55-58 °C (CH₂Cl₂/MeOH 75:25); IR (KBr) ν: 3500-2500 (max at 3243, 3060, 2928, 2854, 2802 N-H,⁺N-H, and C-H st), 1654, 1650, 1633, 1580, 1572, 1522 (C=O, ar-C-C and ar-C-N st) cm⁻¹; HRMS (ESI) calcd for (C₂₈H₄₀N₄O₂+ H⁺) 465.3224, found 465.3212. Elemental analysis, calcd for (C₂₈H₄₀N₄O₂•HCl•1.25H₂O) C 64.10, H 8.55, N 10.68, Cl 6.76; found C 64.04, H 8.54, N 10.08, Cl 7.15.

### Examples 13-15: In vivo studies in APP/PS1 mice

Animals and treatment: The experiments were carried out on male APP/PS1 mice, which express the human mutated forms APPswe and PS1dE9, and wild-type (WT) littermates which were 5 month-old at the beginning of the study. In the present work, identification of transgenic mice was carried out as follows: genomic DNA was isolated from 1-cm tail clips and genotyped by polymerase chain reaction (PCR) technique using the PCR conditions proposed by Jackson Laboratory. Animals were maintained under standard animal housing conditions in a normal 12-h dark-light cycle with free access to food and water. The sample size for experimentation was computed using the Power and Precision software (Biostat, Englewood, NJ, USA), assuming a power of 95% and no missing data. Animal procedures were conducted according to ethical guidelines (European Community Council Directive 86/609/EEC) and approved by the local ethical committee (UB-IDIBELL). The compounds (5 mg/kg) were dissolved in 1% DMSO and 99% saline, and were injected intraperitoneally (i.p.) in a volume of 10 mL/kg body weight. Animals were treated once daily for 4 weeks with (α*S*,7*R*,11*R*)-(Ia), (α*S*,7*S*,11*S*)-(Ia), (±)-(Ib), (±)-(Ic) or the corresponding vehicle (VEH) (wild-type, n = 6; APP/PS1, n = 6, each group). After 2 days of washing period, animals were subjected to behavioral evaluation.

Behavioral evaluation of cognitive performance: The two-object recognition test was performed. This paradigm was performed in a V-maze (Panlab, Barcelona, Spain) because it improves the exploration time of the animals with respect to a classical open field. On day 1, mice were habituated for 9 min, allowing them to freely explore the apparatus. On the second day, mice were placed for 9 min in the maze, where two identical objects were situated at the end of the arms, and the time that the mice spent exploring each object was recorded. Then, 24 h after the training session, animals were placed again in the V-maze where one of the two familiar objects was replaced by a novel object. The time that the animals spent exploring the two objects was recorded and an object recognition index was calculated as the difference between the time spent exploring the novel and the familiar object, divided by the total time spent exploring the two objects. Animals exhibiting memory impairments revealed a lower object recognition index.

Tissue Collection: At the end of the behavioral testing, the animals were sacrificed and their brains were removed. One brain hemisphere was dissected on ice, immediately frozen and stored at -80 °C until processing for protein or RNA quantification. The other brain hemisphere was fixed in 4% paraformaldehyde and processed for immunohistochemistry.

Aβ immunohistochemistry: Tissue samples were embedded in paraffin and coronal sections (4 µm) were cut with a microtome. De-waxed sections were incubated with 98% formic acid (3 min) and then treated with citrate buffer (20 min) to enhance antigenicity. Then, the endogenous peroxidases were blocked by incubation in 10% methanol-1 % H₂O₂ solution (15 min). Sections were blocked with 3% normal horse serum solution and then incubated at 4 °C overnight with the primary antibodies against Aβ (1:50, Dako, Clone 6F/3D). Sections were subsequently rinsed and incubated with biotinylated secondary antibody (Dako), followed by EnVision+ system peroxidase (Dako) and finally with the chromogen diaminobenzidine and H₂O₂. Some sections were incubated without the primary antibody. No immunostaining was detected in these sections. Sections were lightly counterstained with haematoxylin. After staining, the sections were dehydrated and cover-slipped for microscopic observation. The Aβ burden in neocortex was calculated as the percentage of the Aβ deposition area with respect to the total area in 9 representative pictures, corresponding to the main regions where Aβ deposition is observed in APP/PS1 mice. Sections from all the APP/PS1 animals were evaluated by using the Analysis tool of the software Adobe^{®} Photoshop^{®} CS4.

Glial and Aβ double-immunofluorescence: De-waxed sections were stained with a saturated solution of Sudan black B (Merck) for 30 min to block the autofluorescence of lipofuscin granules present in cell bodies, then rinsed in 70% ethanol and washed in distilled water. The sections were treated with 98% formic acid (3 min) and with citrate buffer to enhance antigenicity, and then incubated at 4 °C overnight with combinations of primary antibodies against Aβ (1:50, Dako) and GFAP (1:250, Dako, Denmark) or Iba1 (1:250, Wako, USA). After washing, the sections were incubated with Alexa488 or Alexa546 (1:400, Molecular Probes) fluorescence secondary antibodies against the corresponding host species. After washing, the sections were mounted in Immuno-Fluore Mounting medium (ICN Biomedicals, USA), sealed, and dried overnight. Sections were examined with an Olympus BX51 microscope.

Densitometric quantification of glia around Aβ plaques: Astrocytic and microglial responses to Aβ deposition were evaluated by densitometric quantification of GFAP and Iba1 protein expression levels around Aβ plaques, respectively. The GFAP and Iba1 immunostaining in reference to the Aβ plaque area in 5 representative pictures was taken from the neocortex of each animal (n=5 per group) using the software Adobe^{®} Photoshop^{®} CS4. Statistical analysis: Data were analyzed by two-way ANOVA with genotype and treatment or age as between factors, followed by Tukey's *post hoc* test when required. Aβ and glia quantifications were analyzed by one-way ANOVA followed by Tukey's *post hoc* test when required.

### Example 13: Effects of the compounds on cognitive failure in APP/PS1 mice

APP/PS1 mice treated daily for 4 weeks during early stages of the symptomatic phase (5 months of age) with vehicle, exhibited memory impairment in the two-object recognition test when compared to corresponding wild-type littermates (FIG 1). APP/PS1 mice treated with compounds of formula (α*S*,7*S*,11*S*)-(Ia) and (±)-(Ic) did not exhibit memory impairment. Indeed, the chronic treatment with (±)-(Ic) significantly increased the recognition index of APP/PS1 mice when compared to vehicle-treated animals.

### Example 14: Effects of the compounds on Aβ burden in APP/PS1 mice

Chronic treatment with compounds (α*S*,7*S*,11*S*)-(Ia), (α*S*,7*R*,11*R*)-(Iₐ), (±)-(Ib), and (±)-(Ic) (FIG 2) tends to reduce the Aβ burden in the cortex of APP/PS1 mice when compared to vehicle-treated animals, in particular with the compound (α*S*,7*S*,11*S*)-(Ia) (5 mg/kg/day).

### Example 15: Effects of the compounds on gliosis associated with Aβ deposition in APP/PS1 mice

Double-immunofluorescence revealed that chronic treatment with (α*S*,7*S*,11*S*)-(Ia), (α*S*,7*R*,11*R*)-(Ia), and (±)-(Ib) produced a tendency to reduce the number of astrocytes around Aβ plaques in APP/PS1 mice (FIG 3).The number of microglial cells associated with Aβ plaques was reduced by (α*S*,7*S*,11*S*)-(Ia), (α*S*,7*R*,11*R*)-(Ia), and (±)-(Ic) in comparison to vehicle-treated APP/PS1 animals (FIG 4).

The present observations show that the administration of compounds of formula (α*S*,7*S*,11*S*)-(Ia) and (±)-(Ic) reverse the memory impairment in APP/PS1 transgenic mice when the treatment was started at early symptomatic stages. The positive cognitive effect of (α*S*,7*S*,11*S*)-(Ia) is associated with a reduction in the cortical Aβ plaques deposition and an antiinflammatory effect manifested as decreased microglial reaction in APP/PS1 mice. Both events, Aβ aggregation and inflammation, are early pathological hallmarks occurring in AD, thus suggesting that compounds such as (α*S*,7*S*,11*S*)-(Ia) are promising candidates to reverse or halt the neurodegenerative process leading to dementia.

### REFERENCES CITED IN THE APPLICATION

- A. Cavalli et al., "A Small Molecule Targeting the Multifunctional Nature of Alzheimer's Disease" Angew. Chem. Int. Ed. 2007, vol. 46, pp. 3689-3692)
- WO2007/122274
- WO2011/076969

## Claims

1. A compound of formula (I) or a pharmaceutically acceptable salt thereof, or any stereoisomer or mixture thereof, 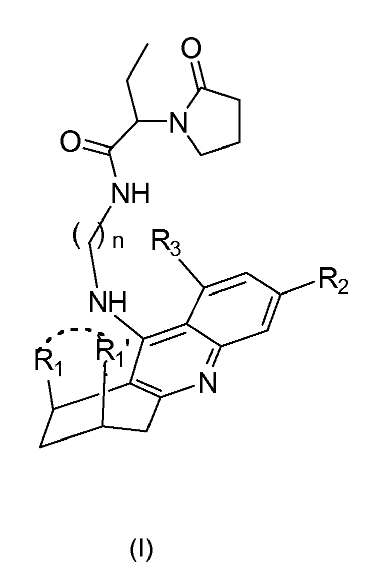 wherein:
R₁ and R₁' are both H or R₁ and R₁' are taken together 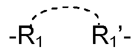 to form biradical of formula -CH₂-C(R₄)=CH-;
R₂ and R₃ are radicals independently selected from the group consisting of H, F, Cl and (C₁-C₄)-alkyl;
R₄ is a (C₁-C₂)-alkyl radical; and
n is an integer from 2 to 15.

2. The compound according to claim 1, wherein R₁ and R₁' are taken together forming a biradical of formula -CH₂-C(R₄)=CH-; wherein R₄ is methyl.

3. The compound according to claim 1, wherein R₁ and R₁' are H.

4. The compound according to any of the claims 1-3, wherein R₂ is Cl or H and R₃ is H.

5. The compound according to any of the claims 1-4, wherein n is an integer from 6 to 8.

6. The compound according to claim 1, which is selected from the following list:
(+)-(α*S*,7*R*,11*R*)-*N*-{7-[(3-chloro-6,7,10,11-tetrahydro-9-methyl-7,11-methanocycloocta[*b*]quinolin-12-yl)amino]heptyl}-2-(2-oxopyrrolidin-1-yl)butanamide;
(-)-(α*S*,7*S*,11*S*)-*N*-{7-[(3-chloro-6,7,10,11-tetrahydro-9-methyl-7,11-methanocycloocta[*b*]quinolin-12-yl)amino]heptyl}-2-(2-oxopyrrolidin-1-yl)butanamide;
(±)-*N*-{7-[(6-chloro-1,2,3,4-tetrahydroacridin-9-yl)amino]heptyl}-2-(2-oxopyrrolidin-1-yl)butanamide; and
(±)-*N*-{7-[(1,2,3,4-tetrahydroacridin-9-yl)amino]heptyl}-2-(2-oxopyrrolidin-1-yl)butanamide.

7. A process for the preparation of the compound of formula (I) as defined in any of the claims 1-6, which comprises:
(a) reacting a compound of formula (II), a salt or a reactive derivative thereof, or any stereoisomer or mixtures thereof, either of the compound of formula (II) or their salts or derivatives, 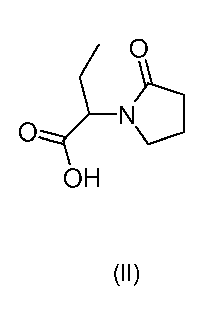 with a compound of formula (III) or a salt thereof, or any stereoisomer or mixture thereof; 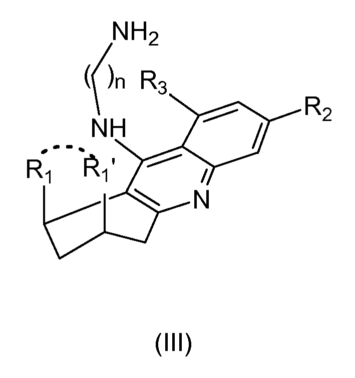 wherein R₁, R₁', R₂, R₃, and n have the same meaning as in any of the claims 1-6; and,
(b) optionally, converting the compound thus obtained into a pharmaceutically acceptable salt by reacting it with a pharmaceutically acceptable acid.

8. The process according to claim 7, further comprising a previous step of submitting a compound of formula (IV) or a salt thereof, or any stereoisomer or mixture thereof, 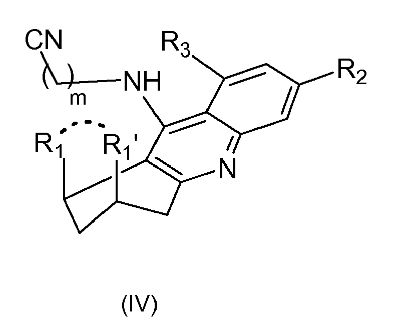 wherein R₁, R₁', R₂, and R₃, have the same meaning as in any of the claims 1-6; and m is an integer from 1 to 14, to a reduction reaction to yield a compound of formula (III) or a salt thereof, or any stereoisomer or mixture thereof.

9. The process according to claim 7, further comprising a previous step of:
(a) reacting a compound of formula (V) or a salt thereof, or any stereoisomer or mixture thereof wherein R_{1,} R₁', R_{2,} and R₃ have the same meaning as in any of the claims 1-6, 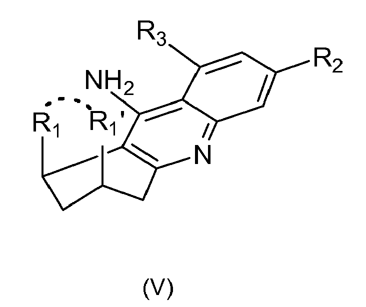 with a compound of formula (VI)
X-(CH₂)ₘ-CN (VI)
wherein m is an integer from 1 to 14 and X is an halogen,
to yield a compound of formula (IV) wherein m is an integer from 1 to 14.

10. The process according to claim 9, further comprising a previous step of:
(a) reacting a compound of formula (VII) or a salt thereof, or any stereoisomer or mixture thereof wherein R₁, R₁', R₂, and R₃, have the same meaning as in any of the claims 1-6, 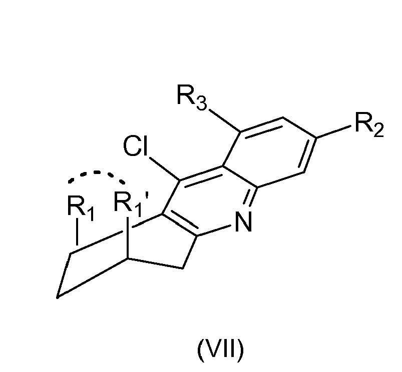 with a compound of formula (VIII)
H₂N-(CH₂)ₙ-NH₂ (VIII)
wherein n is an integer from 2 to 15,
to yield a compound of formula (III) wherein n is an integer from 2 to 15.

11. Compound of formula (I) as defined in any of the claims 1-6, for use as a medicament.

12. Compound of formula (I) as defined in any of the claims 1-6, for use in the prevention and/or treatment of a neurodegenerative disorder.

13. Compound of formula (I) for use as in claim 12, wherein the neurodegenerative disorder is Alzheimer's disease.

14. A pharmaceutical composition comprising a therapeutically effective amount of the compound as defined in any one of the claims 1-6, together with appropriate amounts of one or more pharmaceutically acceptable excipients or carriers.

15. A compound selected from the group consisting of a compound of formula (III) or a salt thereof, or any stereoisomer or mixture thereof, 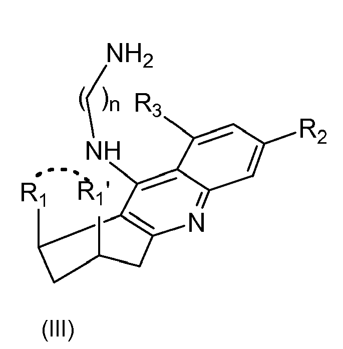 wherein:
R₁ and R₁' are taken together 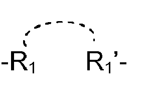
and are biradical of formula -CH₂-C(R₄)=CH-;
R₂ and R₃ are radical independently selected from the group consisting of H, F, Cl and (C₁-C₄)-alkyl;
R₄ is a (C₁-C₂)-alkyl radical; and
n is an integer from 2 to 7; and
a compound of formula (IV) or a salt thereof, or any stereoisomer or mixture thereof, 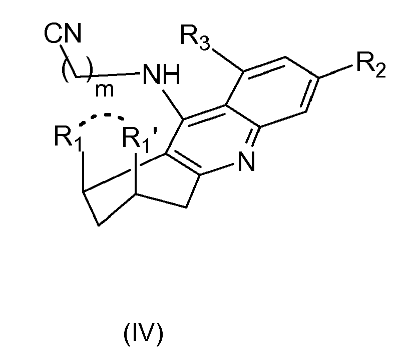
wherein,
both R₁ and R₁' are H;
m is an integer from 1 to 14;
and
R₂ and R₃ are radical independently selected from the group consisting of H, F, Cl and (C₁-C₄)-alkyl;
or, alternatively,
R₁ and R₁' are taken together 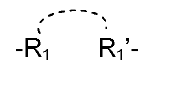
forming a biradical of formula -CH₂-C(R₄)=CH-;
R₂ and R₃ are radical independently selected from the group consisting of H, F, Cl, and (C₁-C₄)-alkyl;
R₄ is a (C₁-C₂)-alkyl radical; and
m is an integer from 1 to 6.
